# EUROPEAN PATENT SPECIFICATION

(11) **EP 0 551 187 B1**
(45) Date of publication and mention of the grant of the patent: **02.08.1995**
(21) Application number: 93300072.1
(22) Date of filing: 06.01.1993
(51) Int. Cl.: A61B 17/58, A61F 2/44

(54) **Apparatus for use in fusion of adjacent spinal bodies**
Vorrichtung zum Immobilisieren angrenzender spinaler Körper
Dispositif pour immobiliser des corps vertébraux adjacents

(30) Priority: 06.01.1992 GB 9200214
(43) Date of publication of application: 14.07.1993
(73) Proprietor: ZIMMER EUROPE LTD, Uxbridge UB10 8PU (GB); Shepperd, John Anthony Norman, Near Battle East Sussex (GB); Bristol-Myers Squibb Company, New York, N.Y. 10154 (US)
(72) Inventor: Petty-Saphon, Satham c/o Zimmer Europe Ltd, Ickenham, Uxbridge UB10 8PU (GB); Shepperd, John Anthony Norman, Near Battle, East Sussex (GB)
(74) Representative: Moir, Michael Christopher

(56) References cited:
- EP-A- 0 260 044
- EP-A- 0 307 241
- FR-A- 2 626 460
- GB-A- 2 207 055
- US-A- 4 401 112
- US-A- 4 743 256
- US-A- 4 905 679

## Description

This invention relates to apparatus for use in fusion of adjacent spinal bodies employing interbody implant means located between disc facing surfaces of anterior parts of said bodies together with means for mutually locating posterior parts of said bodies.

Surgical treatment of lower back pain involves fusion of spinal vertebrae assumed to be responsible for the source of the pain. Such fusion is generally carried out in one of the following ways.
1. Anterior plates are fixed by screws to anterior or medial parts of the vertebral bodies. The term "anterior" here refers to the surgical approach used to access the vertebral bodies. Typically these plates are wider than 20mm and the screws are 5-7mm diameter and 30-50mm in length.
2. Posterior plates fixed to posterior parts of the vertebral bodies by pedicle and/or sacral screws. The term "posterior" here refers to the surgical approach used to access the vertebral bodies.
3. Posterior rods mounted on pedicle and/or sacral screws. These rods typically are 5-7mm diameter and the screws are also 5-7mm diameter and 30-50mm in length.

The posterior plates and screws and the posterior rods and screws are therefore large and require to be implanted through a large skin incision in excess of 10cm. This causes damage to the body stabilising muscular and ligamentous systems. The patient is kept in hospital for many days and return to normal daily life and work is possible only after several months.

The size of the screws in the abovementioned plate/screw, rod/screw systems needs to be as large as 5-7mms because with smaller screw diameters breakage is found to occur. With large screw sizes however implantation needs to be precise as the pedicle is not much larger than the screw itself. Even with these large screw sizes however it has been found that many of the plate/screw and rod/screw systems fail some years after implantation.

The avoidance of such screw failures has been attempted by certain surgeons providing additional support for the anterior parts of the vertebrae through an additional anterior interbody fusion or an additional posterior lumbar interbody fusion (PLIF). Interbody fusion was generally performed with autogenous graft or allograft but this was found to collapse or resolve with time and not contribute to the stability of the posterior system.

The view is also held in some quarters that posterior rod/screw and plate/screw system do not restore appropriately the height of the disc space between affected vertebral bodies causing further degeneration thereof and not relieving pain. Attempts to resolve this problem have resorted to the use of interbody fusion cages in which autogenous bone graft is packed to promote fusion. These cages however have inadequate anchoring means for securing them to the disc facing vertebral plates and can by themselves back out of their location owing to repeated flexure and extension of the spine. They tend also to be insufficiently stable post-operatively giving rise to micromotion which inhibits fusion.

US-A-4743256 discloses the features of the preamble of claim 1.

The prior systems discussed are not satisfactory because they address either the posterior stabilisation need or the anterior stabilisation need or the disc height restoration and anterior stabilisation need. Also, they require the use of large size implant components the installation of which requires open surgery with its concomitant disadvantages.

It is an object of the present invention to provide improved apparatus for use in fusion of adjacent spinal bodies.

The present invention consists in apparatus for use in fusion of adjacent spinal bodies employing rigid interbody implant means located between disc facing surfaces of anterior parts of said bodies together with means for mutually locating posterior parts of said bodies, characterised by said locating means being an elongated link of rod-like section formed with an enlarged eye at one end for engagement with a screw securable to one of said bodies, said elongated link being formed at its other end into means for enabling securing of the link to the other of said bodies, said link being separate from the implant means.

In one form said means for enabling securing of the link to said other of the spinal bodies comprise an end part which extends normal or substantially normal to said elongated part and to the plane of said eye.

The end part may taper at the free end thereof towards said free end.

The plane of said eye may contain the axis of said elongated part.

Alternatively, the plane of said eye is offset from the axis of said elongated part by the thickness of said part.

In another form said elongated part of said link is formed at opposite ends with respective eyes which are substantially coplanar and parallel with said elongated part.

The planes of said eyes may contain the axis of said elongated part, or said elongated part may be formed at opposite ends thereof with respective eyes disposed in parallel or substantially parallel planes spaced apart by the thickness of said elongated part, the plane one of said eyes containing the axis of said elongated part.

In all embodiments of the invention a part at least of said link excluding said eye or eyes thereof may be coated with bioactive material to promote fusion of said part to adjacent bone of one at least of said bodies.

Thus, said elongated part may be coated with bioactive material to promote fusion thereto of adjacent bone of said bodies.

Again in all embodiments, said eye or eyes may be formed with a hardened surface where they engage said screw or an end part extending from an elongated part of a link.

Thus the link may be made from titanium and the hardened surface of the eye or eyes thereof may be provided by titanium nitride.

Said link may be formed from rod of diameter 3 to 4mm and the or each link eye may have a diameter such as to enable close engagement therewith of a pedicle screw shank of 3 to 4mm diameter.

The apparatus may be provided with a pedicle screw formed with a head and a shank adapted to be closely engagable with the or each eye of said link and having an externally threaded part which comprises a substantial part at least of said shank.

The pedicle screw where it engages the eye of a link may be formed with a hardened surface.

Said screw may be formed from titanium and said hardened surface may comprise titanium nitride.

Said links and each of said screws may be dimensioned so as to support in use thereof about half and preferably less than half the spinal load.

Thus, said links and each of said screws may be dimensioned so as to support in use thereof approximately 25 per cent of the spinal load, and perhaps as little as 20% thereof. Such dimensioning permits the links and screws to be employed in microsurgical procedures.

Said interbody implant means may comprise a generally rigid cylindrical body adapted to engage in opposed channels formed in facing end plates of the spinal bodies and having retaining means for fixedly locating said rigid cylindrical body relatively to the spinal bodies, said cylindrical body being of overall dimensions enabling its installation by minimum intervention surgery.

Said retaining means may comprise an external thread (preferably buttress threads) formed on the exterior of said rigid cylindrical body.

Said threads may be interrupted by a plain cylindrical surface of the cylindrical body.

The rigid cylindrical body and its threads may have an overall diameter in the range 5 to 25mm.

Preferably, the overall diameter of said rigid cylindrical body and its threads is in the range 9 to 16mm.

The said interbody implant means and said retaining means thereof may have an exterior surface which is smooth.

The buttress threads may measure between 2 and 3mm from root to tip thereof, and may have a pitch of 3 to 4mm.

Preferably, the buttress thread has a forward facing surface which curves from 60° at the tip of the thread to 0° at the root of the thread relatively to the exterior of said cylindrical body.

Part at least and preferably all of said exterior surface is coated with bioactive material to promote bone ongrowth.

The apparatus of the invention is for use in stabilising the spinal bodies by use of an interbody implant means for stabilising the anterior spinal column and links and pedicle (or sacral) screws which in use stabilise the posterior spinal column. The links and screws support a lesser part, perhaps as little as 20 per cent of the load on the spine. The components of the apparatus thus are able to be provided in sizes suitable for minimum intervention surgery which causes less physical damage to the patient and enables his period in hospital and of rehabilitation to be substantially curtailed.

The invention will now be described, by way of example, with reference to the accompanying, somewhat diagrammatic drawings, in which:-
FIGURE 1 is a side elevation to an enlarged scale of a link according to the invention shown engaged with a pedicle screw;
FIGURES 2 and 3 are an end elevation and plan view to an enlarged scale of the link of Figure 1;
FIGURES 4 and 5 are a front and a side elevation of another form of link according to the invention which can be used;
FIGURE 6 is a side elevation, partly in section, illustrating one form of usage of the apparatus according to Figures 1 to 3;
FIGURE 7 is a plan view of the embodiment of the invention illustrated in Figure 6;
FIGURE 8 is a rear elevation of the apparatus of Figure 6, with certain parts not shown;
FIGURES 9 and 10 are views similar to Figures 7 and 8 but showing the apparatus of the invention in a different position;
FIGURES 11(a) and 11(b) show in side and rear elevation apparatus of the general form of that illustrated in Figures 1 to 3 employed in the fusion of three vertebrae;
FIGURES 12(a) and 12(b) are views generally similar to those of Figures 11(a) and 11(b) but showing the apparatus differently disposed;
FIGURES 13(a) and 13(b) show in side and rear elevation a manner of usage of apparatus generally of the form shown in Figures 1 to 5;
FIGURES 14(a) and 14(b) show the apparatus of Figures 1 to 5 but employed in a different disposition from that of Figure 13;
FIGURE 15 is a side elevation of the interbody implant device forming part of the apparatus of the invention;
FIGURES 16(a), (b) and (c) are respectively a side elevation, an end elevation looking from the left of Figure 16(a) and an end elevation looking from the right of Figure 16(1) illustrating one side of a further embodiment of interbody implant device forming part of the apparatus of the invention, and
FIGURES 17(a), (b) and (c) are views corresponding to Figures 16(a), (b) and (c) of a larger size of interbody implant device than that shown in Figures 16(a), (b) and (c).

Throughout the drawings, like parts have been accorded the same reference numerals.

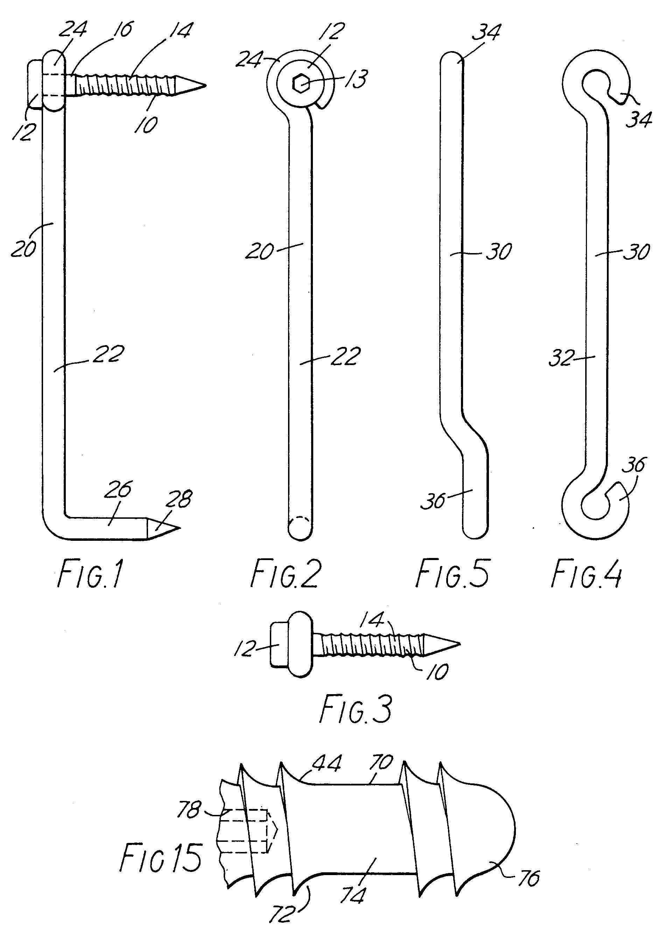

Referring first to Figures 1 to 3, pedicle screw 10 which may, if required for fixing to the sacrum, be a sacral screw, has a head 12 with a cavity 13 of hexagonal cross-section and a shank 14, the latter being externally threaded except for a plain unthreaded part 16 which adjoins the head 12. The cavity 13 is engaged with a tool for manoeuvering the screw 10 to the requisite location during the surgical procedure for installing the posterior spinal column stabilising means. The screw 10 is engaged in a link 20 formed from cylindrical rod of diameter 2 to 5mm and, preferably, about 3mm. The shank of the screw has the same diameter as the rod forming the link 20. The link 20 comprises an elongated rectilinear part 22 at one end of which the rod is bent to form an eye 24, whilst, at the other end of part 22 the rod is formed with an end part 26 which is disposed normal the plane of the eye 24 and at its free end is tapered as at 28. The pedicle screw part 16 is a close fit in the eye 24.

As illustrated the eye 24 is shown to include in its plane the axis of the elongated part 22. It can however be disposed with its plane parallel to the part 22 but spaced from the axis of that part by an amount equal or approximately equal to the thickness of the rod. The spacing of the plane of the eye 24 relatively to the axis of the elongated part 22 can be either on the same side or the opposite side of the part 22 as the end part 26.

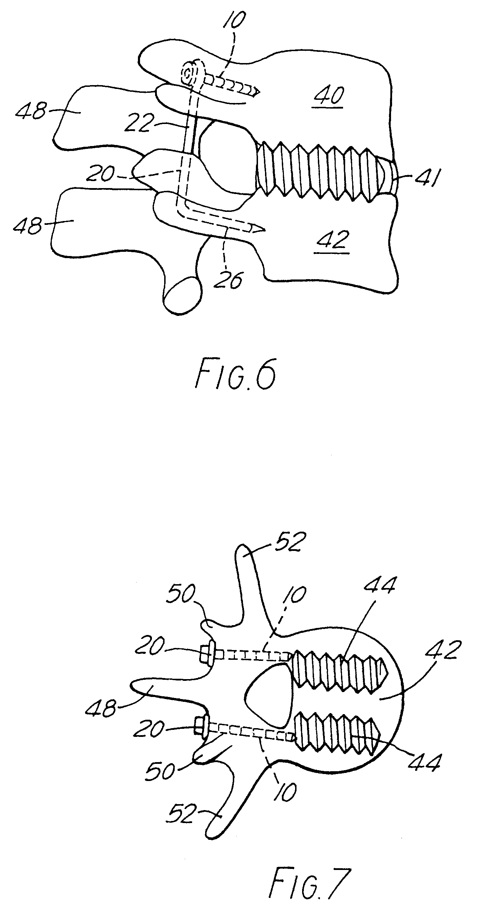

The link 20, as will be apparent, co-operates in use with a single pedicle (or sacral) screw. Another form of link 30 is illustrated in Figures 4 and 5 and is formed from rod of the same diameter as that of link 20 and comprises an elongated rectilinear part 32 bent at its ends to form eyes 34 and 36, the plane of eye 34 containing the axis of part 32 and the plane of eye 36 which is parallel to that of eye 34 being displaced from the axis of part 32 by an amount substantially equal to the thickness of the rod material which is the same as that of link 20. Whilst the plane of the eye 36 has been shown offset with respect to the plane of the eye 34, in alternative forms of the link 30, the eyes 34 and 36 can be coplanar and their common plane can either include the axis of part 32 or be offset from it by an amount substantially equal to the thickness of the rod forming the link. In all its forms, the eyes 34 and 36 of the link 30 are dimensioned to afford a close fit with the unthreaded part 16 of the shank 14 of the pedicle (or sacral) screw 10. Each form of link 20 or 30 excluding the eye or eyes thereof may be partly or entirely coated with a surface layer based on bioactive calcium phosphate ceramic to promote fusion thereto of adjoining bone by bone ongrowth or ingrowth. Alternatively, the surface of the link 20 or 30 excluding the eye or eyes may be porous and also coated with said ceramic.

The eye or eyes of the links are preferably treated to harden the surfaces thereof. Thus in the case of titanium links the surfaces can be treated to form titanium nitride thereon to effect the desired hardening.

The links 20 are provided to accommodate different patients in a range of vertical lengths with 2mm increments. Suitably, the links are made from one of titanium alloy, stainless steel or chrome cobalt.

Referring now to Figures 6 to 8 spinal stabilising apparatus is illustrated for mutually securing adjacent vertebrae 40 and 42 between which disc 41 is located. The apparatus which can be used to secure together any lower thoracic or lumbar vertebrae and with very slight modification, using sacral instead of pedicle screws where screws are placed in the sacrum, could be employed to secure the fifth lumbar vertebra to the sacrum, comprises interbody implant means in the form of a pair of generally cylindrical externally threaded dowels 44 which engage in complementary internally threaded bores formed between the vertebrae 40 and 42. Although two dowels are illustrated, it may be that one centrally disposed dowel only could be used. Various forms of dowel can be employed, e.g. dowels with different forms of thread or without threads but with means for securing the dowels to adjoining vertebrae. When externally threaded dowels are used, they may be provided with additional means for securing the dowels to the adjoining vertebrae. The means for securing the dowels can be either or both mechanical means or biological in nature to provide a surface for promoting fusion thereto of adjoining bone.

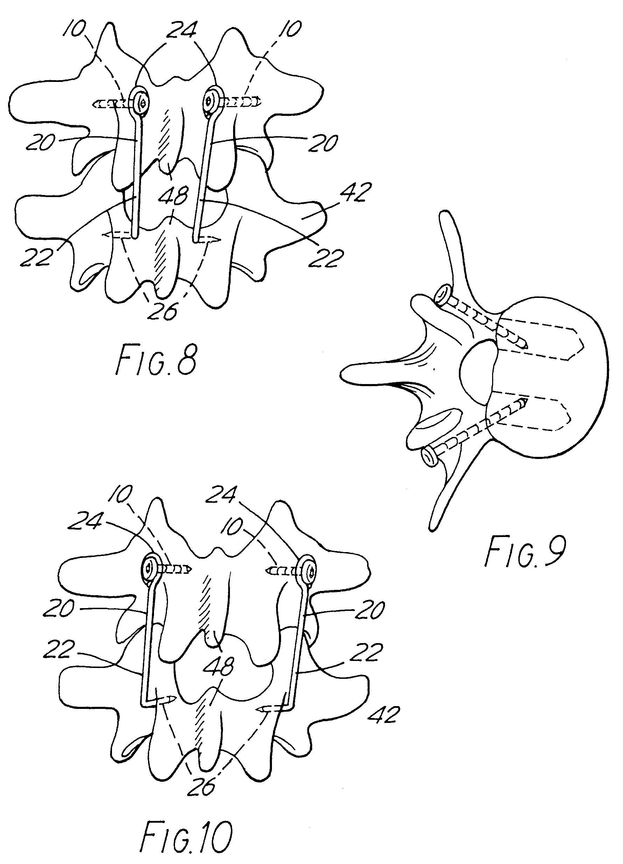

The dowels 44 serve to maintain the desired height between the end plates of the vertebrae 40 and 42 and provide support for anterior parts of the vertebrae 40 and 42 which bear the larger proportion of the load on the spine, the remainder of the load being taken by two bilaterally disposed links 20. These are engaged by pedicle screws 10, the latter in the case of Figures 7 and 8, engaging the pedicles of vertebra 40, each between the spinous process 48 and one of the facet joints 50 whilst the end part 26 of each link 20 is engaged in the vertebra 42 and is also located between the spinous process and the corresponding facet joint 50. In the case of Figures 9 and 10 each of the links 20 is located between a transverse process 52 and the adjacent facet joint 50, the pedicle screws 10 and corresponding end parts 26 of the links being substantially parallel so that the elongated rectilinear link parts 22 are disposed lengthwise of the spine.

Because the greater proportion of the load borne by the spine is supported by the anterior parts of the vertebral bodies, the links 20 and pedicle screws 10 are much lighterweight components than have been used in the case where spinal stabilization has been effected only by fusion of posterior parts of the vertebrae or where, in the past, sharing of the spinal load between anterior implant means and means for mutually securing posterior parts of the immobilized vertebrae has been proposed. Because of the small dimensions of the link it can during surgery be "buried" in the bone surface of the vertebrae 40 and 42 and covered with bone graft to promote fusion.

When a metal cylindrical dowel is employed for the anterior interbody fusion apparatus, the diameter of the body lies in the range 5-25mm, preferably 9-16mm. These size ranges and the fact that the links 20 and screws 10 are of much lighter weight and significantly smaller than previously used posterior column stabilising components, enable the dowel, the links and the screws to be installed by microsurgical procedure which has the advantages of engendering minimal surgical trauma and minimum hospital stay for the patient.

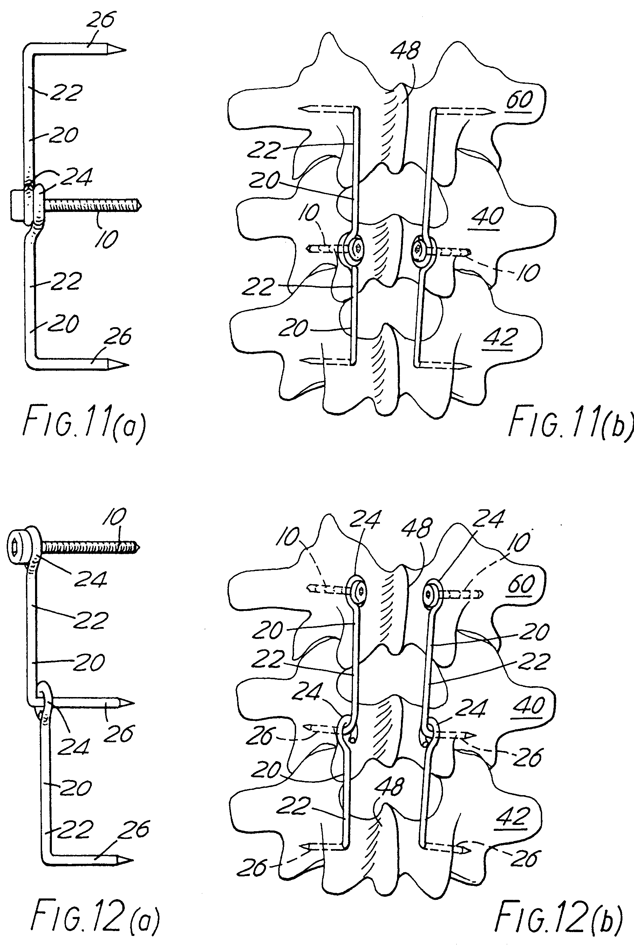

Referring now to the remaining drawings, Figures 11 to 14 illustrate different ways in which the apparatus of the invention may be deployed to effect mutual immobilisation of three vertebrae 40,42 and 60 or of the fourth and fifth lumbar vertebrae and the sacrum although in the following description it is assumed that the third, fourth and fifth lumbar vertebrae are being immobilised.

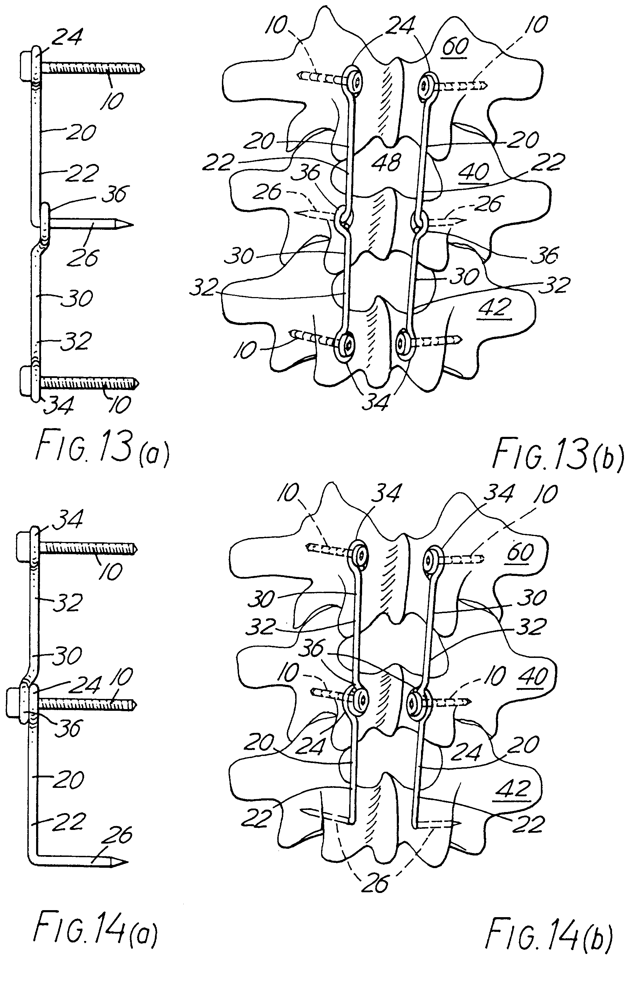

Thus anterior column implant means (not shown) such as pairs of the metal dowels previously referred to are provided at each of the two intervertebral levels whilst pedicle screws 10 and links 20 and, in the case of the arrangements depicted in Figures 13 and 14, also links 30 provide the stabilising means for posterior parts of the vertebrae concerned.

In the case of Figures 11(a) and 11(b) a bilateral arrangement of coupled links 20 is shown wherein on each side of the spinous process 48 are located, in similar positions, to that shown in Figure 8, two links 20 the eyes 24 of which are engaged on pedicle screw 10 which is engaged in the pedicle of vertebra 40 whilst the end parts 26 of the links engage respectively in the vertebrae 42 and 60 and extend generally parallel with the screw 10. It will be observed that the plane of the eye 24 of each lower link 20 is offset from the axis of the elongated part 22 by approximately the diameter of that part and thus enables the parts 22 of each set of upper and lower links to hang collinearly with the parts 22 equidistant from but close to the adjacent vertebrae.

In the arrangement of Figures 12(a) and 12(b) the pedicle screws 10 are each engaged with the eye 24 of one of the associated pair of links 20 and disposed in the pedicle of vertebra 60. The eye 24 of the lower of each pair of links 20 engages the end part 26 of the upper link of the pair and the end parts 26 of the two links engage respectively in the vertebrae 40 and 42 extending therein approximately parallel with the corresponding pedicle screw 10.

In the arrangement of Figures 13(a) and 13(b) there are employed bilaterally two pairs of links each comprising an upper link 20 and a lower link 30. The eye 24 of upper link 20 and the eye 34 of lower link 30 engage on respective screws 10 which are located in respective vertebrae 60 and 42 whilst the eye 36 of the link 30 engages on the end part 26 of link 20 which is located in vertebra 40. The eye 36 is offset from the elongated part 32 of its link 30 so that the parts 22 and 32 of the respective links 20 and 30 are collinearly disposed close to the surfaces of the adjacent vertebrae.

In Figures 14(a) and 14(b) the arrangement differs from that of Figures 13(a) and 13(b) in that the pedicle screws 10 of each set of links 20 and 30 are located in vertebrae 60 and 40 and the link 30 is in the upper position of the pair of links. Also the offset eye 36 of link 30 is located outwardly on the corresponding pedicle screw 10 of the eye 24 of link 20 and the end part 26 is located in the vertebra 42.

Although the arrangements of Figures 11 to 14 are shown in the position corresponding with that of Figures 7 and 8, i.e. with the elongated parts of the pairs of links between the spinous process and the adjacent facet joint they could also be disposed in the position corresponding to that of Figures 9 and 10 in which the elongated parts of the pairs of links are located between an adjacent facet joint and transverse process. Also, the surfaces of the links in these arrangements, as in the earlier arrangements of Figures 6 to 10, excluding the eye or eyes thereof, can be coated with a layer of bioactive material, suitably bioactive calcium phosphate ceramic, to promote bone ingrowth or ongrowth. Alternatively, those surfaces excluding the eye or eyes may be porous and also coated with the said ceramic. Also, in all cases, the eye or eyes of the links can be treated to harden their surfaces, e.g. by forming titanium nitride thereon in the case of titanium links. Surface hardening of the link eyes can also be effected by known techniques of ion implantation. The surface hardening is applied where the eyes engage the pedicle screws 10 or the link parts 26. Similarly the surface of the screws are hardened where they engage the eyes.

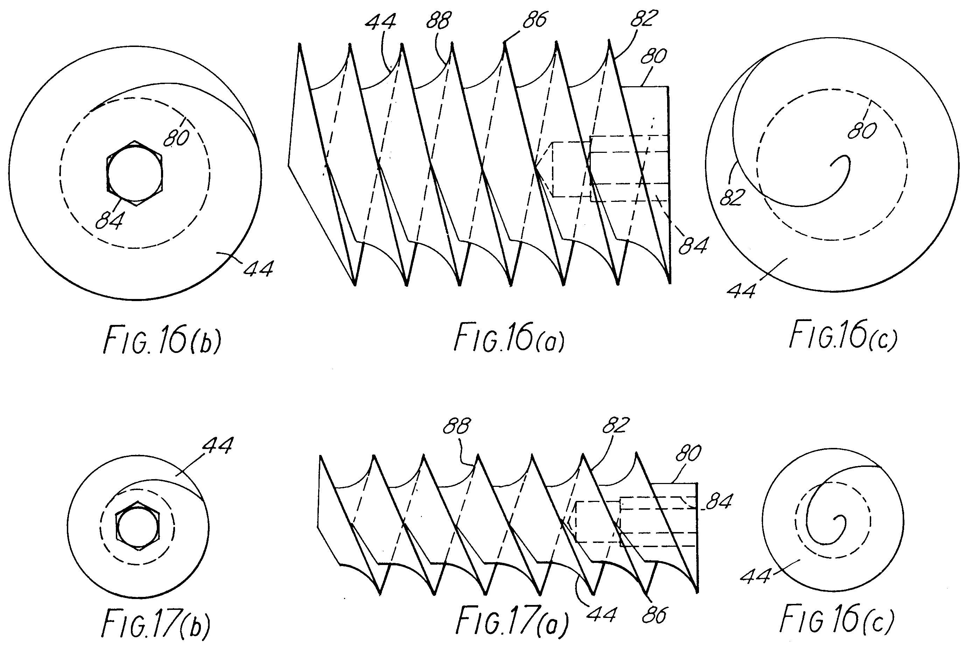

Figure 15 illustrates, to an enlarged scale, a preferred form of the dowel 44 which consists of a generally cylindrical body 70 formed with a buttress thread 72 which is interrupted by a plain cylindrical part 74 of the body. At its forward end the body has a curved nose 76 whilst, at its rear end, there is formed extending axially in the body from one end thereof a blind bore 78 of hexagonal cross-section in which a tool is releasably engaged for guiding the body to and at the location at which it is installed. The entire exterior of the cylindrical body 70 and thread 72 is smooth. The plain cylindrical part 74 of the body or the entire external surface of the body and threads can be coated, as described for the links 20 and 30, to promote bone ingrowth or ongrowth. The buttress thread 72 acts somewhat as a barb which, when the dowel is installed, inhibits rearward movement thereof which repeated flexure of the spine may otherwise occasion.

Referring now to Figures 16(a), (b) and (c) and 17(a), (b) and (c) which show two sizes of an alternative and preferred form of metal, interbody dowel to that of Figure 15: in this form the dowel is formed on a cylindrical core 80 thereof with a continuous external buttress thread 82. Again, the exterior surface of the core and thread is smooth and may be coated with bioactive material to promote bone ongrowth or ingrowth. Also, as in the case of the embodiment of Figure 15, an axially extending blind bore 84 of hexagonal cross-section is provided in which a tool is releasably engaged for guiding the dowel to and at the location at which it is installed.

The dowel of Figures 16(a), (b) and (c) has a core of diameter 10mm and a thread of overall diameter 16mm so that the turns of the thread extend from root to tip thereof by 3mm from the surface of the core. The thread pitch is 3.5mm measured between corresponding radial locations at outer edge 86 of the thread. The forward surface 88 of the thread slopes at 60° to the axis of the core at the thread edge 86 and curves to zero inclination at the core diameter.

In the embodiment of Figures 17(a), (b) and (c) the core diameter is 5mm and the overall diameter of the thread is 9mm thus providing a thread height from root to tip thereof of 2mm. The thread pitch is again 3.5mm measured as before and the slope of the forward surface 88 is again inclined at 60° to the axis of the core at the edge 86 of the thread and curves to zero inclination at the surface of the core.

Smaller and larger sizes of the dowel 44 than those described as well as intermediate sizes can be provided though in the preferred range of sizes the core will be between 5 and 10mm diameter and the overall diameter of the threads will be between 9 and 16mm diameter and the thread pitch will be between 3 and 4mms. The buttress form of the thread acts, as in the case of the embodiment of Figure 15, as a barb inhibiting extrusion, that is to say backing out motion, of the dowel.

The design of the forms of dowel 44 illustrated in Figures 15 and more particularly in Figures 16(a), (b) and (c) and 17(a), (b) and (c) provides a novel thread design for metal interbody implants the pitch and height of the thread turns affording good bone purchase and a large surface for bone ongrowth and, therefore, interbody fusion. Because of the large surface of the thread, the spinal loading stress on the dowel is optimally distributed. Moreover, the sizes referred to of the dowel can be employed in minimum intervention surgical procedures which substantially diminish tissue damage and thus make for substantially shorter patient stay in hospital and recuperation periods.

Although the links 20 and 30 have been described as being of lightweight construction because they are required to support a lesser proportion of the spinal load, they can be used in more substantial form if required and in general they afford together with the associated pedicle screws a novel and advantageous means of support for the posterior spinal column.

## Claims

1. Apparatus for use in fusion of adjacent spinal bodies employing rigid interbody implant means (44) located between disc facing surfaces of anterior parts of said bodies together with means for mutually locating posterior parts of said bodies, characterised by said locating means being an elongated link (20) of rod-like section formed with an enlarged eye (24) at one end for engagement with a screw (10) securable to one of said bodies, said elongated link being formed at its other end into means (26 or 36) for enabling securing of the link to the other of said bodies, said link being separate from the implant means (44).

2. Apparatus as claimed in Claim 1, characterised in that said means for enabling securing of the link (20) to said other of the spinal bodies comprise an end part (26) which extends normal or substantially normal to the remainder of the link and to the plane of said eye.

3. Apparatus as claimed in Claim 2, characterised in that said end part tapers at the free end (28) thereof towards said free end.

4. Apparatus as claimed in Claim 1, characterised in that said elongated link is formed at opposite ends with respective enlarged eyes (34,36) which are substantially coplanar and parallel with said elongated link.

5. Apparatus as claimed in Claim 2, 3 or 4, characterised in that the axis of said elongated link (20) lies in a plane of a said eye or eyes (34).

6. Apparatus as claimed in Claim 2, 3 or 4, characterised in that the plane of a said eye (36) is offset from the axis of said elongated link (20) by the thickness of said link.

7. Apparatus as claimed in any preceding claim, characterised in that a part at least of said link (20) excluding said eye or eyes thereof is coated with bioactive material to promote fusion of said part to adjacent bone of one at least of said bodies.

8. Apparatus as claimed in any preceding claim, characterised by a screw (10) formed with a head and a shank adapted to be closely engageable with the or each eye of said link and having an externally threaded part (14) which comprises a substantial part at least of said shank.

9. Apparatus as claimed in any preceding claim, characterised in that said links and each of said screws is dimensioned so as to support in use thereof less than half the spinal load.

10. Apparatus as claimed in any preceding claim, characterised in that said interbody implant means (44) comprises a generally rigid cylindrical body adapted to engage in opposed channels formed in facing end plates of the spinal bodies and having retaining means (72) for fixedly locating said rigid cylindrical body relatively to the spinal bodies, said cylindrical body being of overall dimensions enabling its installation by minimum intervention surgery.

11. Apparatus as claimed in Claim 10, characterised in that said interbody implant means and said retaining means thereof have an exterior surface which is smooth, at least part of said exterior surface is coated with bioactive material to promote bone ongrowth.

12. Apparatus as claimed in Claim 10 or 11, characterised in that said retaining means comprise an external thread (72) formed on the exterior of said rigid cylindrical body.

13. Apparatus as claimed in Claim 12, characterised in that the external thread is a buttress (82) thread with a sloped forward surface (88) directed to the nose of the rigid cylindrical body.

## Patentansprüche

1. Vorrichtung zur Verwendung bei der festen Verbindung bzw. Fusion benachbarter Wirbelsäulenkörper unter Verwendung starrer, zwischen den Körpern angeordneter Implantatmittel (44), die zwischen den Bandscheiben zugewandten Oberflächen vorderer Teile der genannten Körper angeordnet sind, zusammen mit Mitteln zur gegenseitigen Festlegung hinterer Teile der genannten Körper, dadurch **gekennzeichnet**, daß die genannten Festlegungsmittel ein längliches Verbindungsglied (20) von stabartigem Querschnitt sind, das mit einem vergrößerten Auge (24) am einen Ende zum Eingriff mit einer Schraube (10) ausgebildet ist, die an einem der genannten Körper befestigbar ist, wobei das genannte, längliche Verbindungsglied an seinem anderen Ende als Mittel (26 oder 36) zum Ermöglichen der Befestigung des Verbindungsgliedes am anderen der genannten Körper ausgebildet ist, wobei das genannte Verbindungsglied von den Implantatmitteln (44) getrennt ist.

2. Vorrichtung wie beansprucht in Anspruch 1, dadurch **gekennzeichnet**, daß die genannten Mittel zum Ermöglichen der Befestigung des Verbindungsgliedes (20) am anderen der Wirbelsäulenkörper ein Endteil (26) umfassen, das sich senkrecht oder im wesentlichen senkrecht zum Rest des Verbindungsgliedes und zur Ebene des genannten Auges erstreckt.

3. Vorrichtung wie beansprucht in Anspruch 2, dadurch **gekennzeichnet**, daß das genannte Endteil sich an seinem freien Ende (28) zum genannten freien Ende hin verjüngt.

4. Vorrichtung wie beansprucht in Anspruch 1, dadurch **gekennzeichnet**, daß das genannte längliche Verbindungsglied an gegenüberliegenden Enden mit jeweiligen vergrößerten Augen (34, 36) ausgebildet ist, die im wesentlichen koplanar und parallel zum genannten länglichen Verbindungsglied verlaufen.

5. Vorrichtung wie beansprucht in Anspruch 2, 3 oder 4, dadurch **gekennzeichnet**, daß die Achse des genannten länglichen Verbindungsgliedes (20) in einer Ebene des genannten Auges oder der genannten Augen (34) liegt.

6. Vorrichtung wie beansprucht in Anspruch 2, 3 oder 4, dadurch **gekennzeichnet**, daß die Ebene eines genannten Auges (36) gegenüber der Achse des genannten länglichen Verbindungsgliedes (20) um die Dicke des genannten Verbindungsgliedes versetzt ist.

7. Vorrichtung wie beansprucht in irgendeinem vorangehenden Anspruch, dadurch **gekennzeichnet**, daß ein Teil mindestens des genannten Verbindungsgliedes (20), der dessen genanntes Auge oder genannte Augen ausschließt, mit bioaktivem Material beschichtet ist, um die feste Verbindung bzw. Fusion des genannten Teils mit dem benachbarten Knochen mindestens eines der genannten Körper zu fördern.

8. Vorrichtung wie beansprucht in irgendeinem vorangehenden Anspruch, **gekennzeichnet** durch eine Schraube (10), die mit einem Kopf und einem Schaft ausgebildet ist, der dazu eingerichtet ist, in engen Eingriff mit dem oder jedem Auge des genannten Verbindungsgliedes gebracht zu werden und der einen mit einem Außengewinde versehenen Teil (14) aufweist, der mindestens einen wesentlichen Teil des genannten Schaftes umfaßt.

9. Vorrichtung wie beansprucht in irgendeinem vorangehenden Anspruch, dadurch **gekennzeichnet**, daß die genannten Verbindungsglieder und jede der genannten Schrauben so bemessen sind, daß sie in ihrem Gebrauch weniger als die Hälfte der Wirbelsäulenlast tragen.

10. Vorrichtung wie beansprucht in irgendeinem vorangehenden Anspruch, dadurch **gekennzeichnet**, daß die genannten, zwischen den Körpern angeordneten Implantatmittel (44) einen insgesamt starren, zylindrischen Körper umfassen, der dazu eingerichtet ist, in gegenüberliegende Kanäle einzugreifen, die in zugewandten Endplatten der Wirbelsäulenkörper ausgebildet sind, und der Haltemittel (72) zum festen Anordnen des genannten starren zylindrischen Körpers relativ zu den Wirbelsäulenkörpern aufweist, wobei der genannte zylindrische Körper eine Gesamtabmessung aufweist, die seine Anbringung durch einen minimalen chirurgischen Eingriff ermöglicht.

11. Vorrichtung wie beansprucht in Anspruch 10, dadurch **gekennzeichnet**, daß die zwischen den Körpern angeordneten Implantatmittel und deren genannte Haltemittel eine Außenoberfläche aufweisen, die glatt ist, wobei mindestens ein Teil der genannten Außenoberfläche mit bioaktivem Material beschichtet ist, um das Anwachsen des Knochens zu fördern.

12. Vorrichtung wie beansprucht in Anspruch 10 oder 11, dadurch **gekennzeichnet**, daß die Haltemittel ein Außengewinde (72) aufweisen, das an der Außenseite des genannten starren zylindrischen Körpers ausgebildet ist.

13. Vorrichtung wie beansprucht in Anspruch 12, dadurch **gekennzeichnet**, daß das Außengewinde ein Sägezahngewinde (82) mit einer geneigten vorderen Fläche (88) ist, die der Nase des starren zylindrischen Körpers zugewandt ist.

## Revendications

1. Appareil utilisable pour l'immobilisation de corps vertébraux adjacents, employant un dispositif d'implant rigide intercorps (44) placé entre des surfaces de disque en regard présentées par les parties antérieures des dits corps, ainsi qu'un dispositif de positionnement mutuel des parties postérieures desdits corps, caractérisé en ce que lesdits moyens de positionnement sont une pièce de liaison allongée (20) en forme de tige à une extrémité de laquelle est formé un oeillet agrandi (24) pour recevoir une vis (10) pouvant être fixée à un desdits corps, l'autre extrémité de ladite pièce de liaison allongée étant formée en un élément (26 ou 36) qui permet de fixer la pièce de liaison à l'autre desdits corps, ladite pièce de liaison étant séparée du dispositif d'implant (44).

2. Appareil suivant la revendication 1, caractérisé en ce que ledit élément permettant la fixation de la pièce de liaison (20) audit autre des corps vertébraux comprend une partie d'extrémité (26) qui est perpendiculaire ou sensiblement perpendiculaire au reste de la pièce de liaison et au plan dudit oeillet.

3. Appareil suivant la revendication 2, caractérisé en ce que ladite partie d'extrémité est effilée, à son extrémité libre (28), vers ladite extrémité libre.

4. Appareil suivant la revendication 1, caractérisé en ce que ladite pièce de liaison allongée présente, à ses extrémités opposées, des oeillets agrandis respectifs (34,36) qui sont sensiblement dans un même plan et parallèles à ladite pièce de liaison allongée.

5. Appareil suivant la revendication 2,3 ou 4, caractérisé en ce que l'axe de ladite pièce de liaison allongée (20) est situé dans un plan d'un dit oeillet ou des dits oeillets (34).

6. Appareil suivant la revendication 2,3 ou 4, caractérisé en ce que le plan d'undit oeillet (36) est décalé de l'axe de ladite pièce de liaison allongée (20), d'une quantité égale à l'épaisseur de ladite pièce de liaison.

7. Appareil suivant une quelconque des revendications précédentes, caractérisé en ce qu'une partie au moins de ladite pièce de liaison (20), à l'exclusion de sondit oeillet ou de sesdits oeillets, est revêtue avec une matière bioactive pour favoriser la fusion de ladite partie à l'os adjacent d'au moins un desdits corps.

8. Appareil suivant une quelconque des revendications précédentes, caractérisé en ce qu'il comprend une vis (10) ayant une tête et une queue prévus pour venir étroitement en prise avec l'oeillet ou chaque oeillet de la dite pièce de liaison et ayant une partie extérieurement filetée (14) qui constitue une partie substantielle au moins de ladite queue.

9. Appareil suivant une quelconque des revendications précédentes, caractérisé en ce que lesdites pièces de liaison et chacune desdites vis sont dimensionnées de façon à supporter, lors de leur utilisation, moins de la moitié de la charge vertébrale.

10. Appareil suivant une quelconque des revendications précédentes, caractérisé en ce que ledit dispositif d'implant intercorps (44) comprend une pièce cylindrique sensiblement rigide prévue pour s'engager dans des canaux opposés formés dans les plaques d'extrémité en regard des corps vertébraux, et comportant des moyens de retenue (72) pour positionner de façon fixe ladite pièce cylindrique rigide par rapport aux corps vertébraux, ladite pièce cylindrique ayant des dimensions globales qui permettent son installation avec une intervention chirurgicale minimale.

11. Appareil suivant la revendication 10, caractérisé en ce que ledit dispositif d'implant intercorps et ses moyens de retenue ont une surface extérieure qui est lisse, et au moins une partie de ladite surface extérieure est revêtue avec une matière bioactive pour favoriser l'incorporation de l'os.

12. Appareil suivant la revendication 10 ou 11, caractérisé en ce que lesdits moyens de retenue comprennent un filetage extérieur (72) formé sur l'extérieur de ladite pièce cylindrique rigide.

13. Appareil suivant la revendication 12, caractérisé en ce que le filetage extérieur est un filetage trapézoïdal (82) ayant une surface avant inclinée (88) dirigée vers le nez de la pièce cylindrique rigide.
